# EUROPEAN PATENT APPLICATION

(11) **EP 2 578 222 A1**
(43) Date of publication of application: **10.04.2013**
(21) Application number: 12006962.0
(22) Date of filing: 08.10.2012
(51) Int. Cl.: A61K 36/53

(54) **Plant extract composition**

(30) Priority: 06.10.2011 EP 11008106
(71) Applicant: Lanwehr, Christoph Alexander Bernhard, 58454 Witten (DE)
(72) Inventor: Lanwehr, Christoph Alexander Bernhard, 58454 Witten (DE); Parlar, Harun, 85406 Zolling (DE)
(74) Representative: Hartz, Nikolai

(57) **Abstract**

Process for the preparation of a plant spumate composition, comprising
(a) soaking plant material in water for a predetermined amount of time at a temperature of from 40°C to 100°C for obtaining an aqueous mixture containing a plant extract and extracted plant material;
(b) generating a turbulent flow condition in the aqueous mixture containing a plant extract at a temperature of from 5°C to 50°C in the presence of a gaseous fluid for obtaining a foam layer containing a plant foam extract;
(c) separating the foam layer from the aqueous mixture; and
(d) drying the separated foam layer for obtaining a plant spumate composition.

## Description

### Field of the Invention

The present invention relates to a process for the preparation of a plant spumate composition. Moreover, the present invention relates to a plant spumate composition obtainable by the process of the present invention. Moreover, the present invention relates to a cosmetic composition containing the plant spumate composition according to the present invention. Finally, the present invention relates to a use of the plant spumate composition according to the present invention for the preparation of a cosmetic composition.

The process of the present invention provides novel plant extract compositions having high content of active agents providing properties such as antioxidant properties, surfactant properties, emulsifier properties and/or pharmacological properties, which are useful in cosmetic compositions.

### Background of the Invention

Processes for the preparation of herbal spumate compositions are known. Backleh M, Leupold G, and Parlar H. disclose in J. Agric. Food Chem. 2003, 51(5), 1297-1301 a method for extracting and separating components of rosemary by using an adsorptive bubble separation (ABS) technique including a foaming step. Accordingly, rosemary plant material is extracted with an aqueous medium and the aqueous medium containing the rosemary extract is treated with a flow of gas such as nitrogen in order to generate a foam which is allowed to rise in a column above the aqueous medium. The foaming is continued for an extended period of time so that a separation of the components occurs along the height of the foam column.

ABS is based on the enrichment of surface active components contained in the aqueous mixture at the interface between the gaseous and the liquid phase. The separation of different components by ABS is based on stability differences of the foam bubbles in a system wherein foam is continuously generated and collapsed. Accordingly, foam bubbles containing components forming unstable foams collapse more rapidly and remain closer to the lower portion of the foam than foam bubbles containing components forming stable foams which are enriched at the upper portions of the foam layer.

The ABS process cannot easily be used for the large scale preparation of a plant extract containing a specific mixture of valuable components for use in cosmetic preparations. Moreover, there is a need for novel spumate compositions having novel combinations of components and properties.

### Summary of the Invention

Therefore, it is the problem of the present invention to provide a process for the preparation of a plant spumate composition, which may be carried out on an industrial scale and which provides a novel composition in high yield and without deterioration of the oxidizable components contained in the composition, which has useful properties in the preparation of a cosmetic composition.

Moreover, it is the problem of the present invention to provide a plant spumate composition having novel properties and components.

Moreover, it is the problem o the present invention to provide a cosmetic composition containing a plant extract and having high storage stability.

Finally, it is the problem of the present invention to provide a specific use of the plant spumate composition according to the present invention.

The present invention provides a process for the preparation of a plant spumate composition, comprising
(a) soaking plant material in water for a predetermined amount of time at a temperature of from 40°C to 100°C for obtaining an aqueous mixture containing a plant extract and extracted plant material;
(b) generating a turbulent flow condition in the aqueous mixture containing a plant extract at a temperature of from 5°C to 50°C in the presence of a gaseous fluid for obtaining a foam layer containing a plant foam extract;
(c) separating the foam layer from the aqueous mixture; and
(d) drying the separated foam layer for obtaining a plant spumate composition.

The present invention also provides a plant spumate composition obtainable by the process of the present invention.

Furthermore, the present invention provides a cosmetic composition containing the plant spumate composition according to the present invention.

Finally, the present invention provides a use of the plant spumate composition for the preparation of a cosmetic composition.

The present invention is based on the recognition that by foaming of an aqueous plant extract in a step wherein a high amount of energy is introduced into the aqueous plant extract by a turbulent flow in the presence of a gaseous fluid, a composition of the plant spumate may be obtained which is useful for the preparation of a cosmetic composition. Accordingly, the present invention provides a process which requires water as the only reagent whereby the selection and separation of the plant spumate composition is accomplished by using physical measures using the properties of the components of the plant extract contained in the aqueous mixture.

Due to the rough conditions during foaming and in the absence of a resolution of the components across a foam layer, the plant spumate composition obtainable according to the process of the present invention contains surface active components and valuable components which are not surfactants. The specific composition may be advantageously used for the preparation of a cosmetic composition.

The primary extract obtained by the present invention may be further purified by ABS whereby the desired components are obtained in a yield which is higher as compared to the case of using ABS alone.

### Brief description of the Figures

Figure 1 shows a device for generating a turbulent flow condition in an aqueous mixture containing a plant extract at a temperature of from 5°C to 50°C in the presence of a gaseous fluid for obtaining a foam layer containing a plant foam extract.
Figure 2 shows a TIC Headspace-SPME-GC-MS spectrum of rosemary spumate according to the present invention.
Figure 3 shows a TIC Headspace-SPME-GC-MS spectrum of commercial dried and cut rosemary (Fa. Fuchs).
Figure 4 shows a TIC Headspace-SPME-GC-MS spectrum of freshly cut rosemary.

### Detailed Description of the preferred Embodiments

For the purpose of the present invention, a spumate composition is a composition which is obtainable based on a foam. The foam may be obtained by a turbulent flow of an aqueous mixture containing surface active agents. Surface active agents suitable for forming a foam are contained in an aqueous plant extract. According to the present invention it is preferred that only surface active agents contained in an aqueous plant extract are used for providing a foam. The foam may be separated from the aqueous mixture. Residual moisture in the foam may be eliminated by drying whereby a dried spumate composition is obtained. According to the present invention, the plant spumate composition is at least partially dried in order to provide a product which may be conveniently stored and handled.

According to the present invention, the foaming step as such represents a selection and separation step providing a spumate composition which is different from the composition of the plant extract contained in the aqueous mixture used for generating the foam. The approach according to the present invention is fundamentally different from conventional ABS, wherein separation of components depends on the establishment of thermodynamic equilibrium reflecting the partition coefficient of different components. Rather, according to the present invention a defined no equilibrium state is generated based on the introduction of mechanical energy into the aqueous mixture containing a plant extract by a turbulent flow whereby the non-equilibrium state is quenched by the separation of the foam from the aqueous mixture.

The spumate composition of the present invention contains surface active agents. Moreover, the spumate composition of the present invention may contain further components which are not to be considered as surface active agents, but which are contained in the foam formed by the surface active agents.

According to a preferred embodiment, the spumate composition contains at least 90 wt.%, more preferably 95 wt.%, even more preferably 99 wt.% of organic compounds which are extracted from the plant material used for the preparation of the plant spumate composition of the present invention. Given that the process of the present invention may be carried out without any auxiliary organic compounds, it is possible to provide a plant spumate composition which only contains organic compounds extracted from the plant material whereby traces of organic solvents or other additives may be avoided.

The plant spumate composition of the present invention may contain residual water. The amount of the residual water is preferably at most 10, more preferably at most 5 and even more preferably 1%.

The process of the invention comprises a step of soaking plant material in water for a predetermined amount of time at a temperature of from 40°C to 80°C for obtaining an aqueous mixture containing a plant extract and extracted plant material.

The plant material may preferably be selected from rosemary (Rosmarinus officialis) containing carnosic acid((4aR,10aS)-5,6-dihydroxy-1,1-dimethyl-7-propan-2-yl-2,3,4,9,10,10a-hexahydrophenanthrene-4a-carboxylic acid); Hypericum (St John's wort) containing hyperforin ((1 R,5S,7R,8S)-4-hydroxy-8-methyl-3,5,7-tris(3-methylbut-2-enyl)-8-(4-methylpent-3-enyl)-1-(2-methylpropanoyl)bicyclo[3.3.1]non-3-ene-2,9-dione); chili pepper and other plants of the genus Capsicum containing capsaicin (8-Methyl-N-vanillyl-trans-6-nonenamide); ginger containing gingerole ((S)-5-hydroxy-1-(4-hydroxy-3-methoxyphenyl)-3-decanone); hops containing humulone ((6R)-3,5,6-Trihydroxy-2-(3-methylbutanoyl)-4,6-bis(3-methylbut-2-en-1-yl)cyclohexa-2,4-dien-1-one); orange (Citrus sinensis) containing xanthophylls including luteine, zeaxanthine, neoxanthine, violaxanthine, and α- and β-cryptoxanthin; carrots (Daucus carota subsp. Sativus) containing carotene; curcuma containing curcuminoide (1E,6E)-1,7-bis (4-hydroxy-3-methoxyphenyl) -1,6-heptadiene-3,5-dione; Potato fruits (Solanum tuberosum)containing solanine; kava plant containing Flavokavin (A: (E)-3-(4-methoxyphenyl)-1-(2,4-bismethoxy-6-hydroxyphenyl)propenone, B: (E)-1-(2-Hydroxy-4,6-dimethoxy-phenyl)-3-phenyl-propenone, C: (E)-1-(2-Hydroxy-4,6-dimethoxy-phenyl)-3-(4-hydroxy-phenyl)-propenone); plants of the genus Coffea containing chlorogenic acid (1S,3R,4R,5R)-3-{[(2Z)-3-(3,4-dihydroxyphenyl)prop-2-enoyl]oxy}-1,4,5-trihydroxycyclohexanecarboxylic acid; plants of the genus Sesamum containing sesamole (3,4-Methylenedioxyphenol); plants of the family Piperaceae such as black pepper (Piper nigrum) which contain piperine (1-[5-(1,3-benzodioxol-5-yl)-1-oxo-2,4-pentadienyl]piperidine); Aloe vera containing aloin (e.g. (10S)-10-Glucopyranosyl-1,8-dihydroxy-3-(hydroxymethyl)-9(10H)-anthracenone); onion (Allium cepa) containing isoalliin; and Prunus dulcis containing amygdalin [(6-O-β-D-glucopyranosyl-β-D-glucopyranosyl)oxy](phenyl)acetonitrile.

In general, the plant material may include plant leaves, fruits, and/or flowers. Roots and stems of a plant may also be used in case the desired components are contained in such plant material as for example in ginger. More preferably, the plant material consists essentially of plant leaves, fruits, and/or flowers. The plant leaves, fruits and/or flowers may preferably be used as such. However, it is possible to further comminute the plant leaves and/or flowers. The plant material may be dried. However, it is preferable that the plant material is used when it is freshly harvested.

Preferably, in case of a herbal extract of rosemary, the plant material includes plant leaves, and/or flowers. More preferably, the plant material consists essentially of plant leaves and/or flowers. The plant leaves and/or flowers may preferably be used as such. However, it is possible to further comminute the plant leaves and/or flowers. The plant material may be dried. However, it is preferable that the plant material is used when it is freshly harvested.

According to a specific embodiment, the plant material is rosemary (Rosmarinus officinalis). As a preferred plant material, rosemary having a content of carnosic acid of at least 260 mg/kg, preferably at least 280 mg/kg may be mentioned.

According to a further specific embodiment, the plant material is leafs, flowers and/or fruits of Hypericum perforatum (St John's wort).

According to a further specific embodiment, the plant material is fruits of chili peppers.

According to a still further embodiment, the plant material is the rhizome of the plant Zingiber officinale (ginger).

According to a still further specific embodiment, the plant material is the female flower clusters (seed cones or strobiles) of a hop species (Humulus lupulus).

According to a further specific embodiment, the plant material is leafs, flowers and/or fruits of Citrus sinensis.

According to a further specific embodiment, the plant material is roots of carrots (Daucus carota subsp. Sativus).

According to a further specific embodiment, the plant material is fruits of a plant of the plant family Zingiberaceae Curcuminoide such as curcuma.

According to a further specific embodiment, the plant material is fruits of potato plants (Solanum tuberosum).

According to a still further specific embodiment, the plant material is leafs and fruits of Kava-Kava.

According to a further specific embodiment, the plant material is coffee beans.

According to a further specific embodiment, the plant material is seeds of sesame (Sesamum indicum).

According to a further specific embodiment, the plant material is fruits of black pepper (Piper nigrum).

According to a further specific embodiment, the plant material is leafs of Aloe vera.

According to a further specific embodiment, the plant material is leafs and roots of onions (allium cepa).

According to a further specific embodiment, the plant material is bitter almonds (seeds of the tree Prunus dulcis).

In the soaking step, the soaking time is not particularly limited and may be selected from a range of from 1 minute to 120 minutes. Preferably, the soaking is carried out for 20 to 90 minutes, more preferably 40 to 70 minutes. If the soaking time is below 1 minute, the extraction efficiency of the soaking step may be deteriorated. If the soaking time is excessively long, then there is a danger that sensitive compounds in the plant extract may be deteriorated.

The soaking temperature is in a range of from 40 °C to 80 °C. Preferably, the soaking temperature is in the range of from 50 to 70 °C. If the soaking time is below 40 °C, the extraction efficiency of the soaking step may be deteriorated. If the soaking temperature is excessively high, there is a danger that sensitive compounds in the plant extract may be deteriorated.

The plant material is preferably soaked in water such as tab water. The water preferably has a pH in the range of from 5 to 9, more preferably from 6 to 8. In a specific embodiment, the pH of the water is in the range of from 6.5 to 7.5, preferably about 7.0.

The pH of the mixture may be adjusted by the addition of an acid or a base. Suitable acids may be selected from organic or inorganic acids. Examples of organic acids are acetic acid and citric acid. Examples of inorganic acids are phosphoric acid, sulfuric acid and hydrochloric acid. Suitable bases may be selected from organic and inorganic bases. Examples of an organic base are pyridine and triethyl amine. Examples of inorganic bases are sodium hydrogen carbonate, ammonium hydroxide, sodium hydroxide and potassium hydroxide.

The amount of water is not particularly limited. However, it is preferred that water is used in an amount of from 10 to 400 parts per 10 part by weight of the plant material. Preferably, the amount of water is in the range of from 40 to 100 parts by weight per 10 parts by weight of the plant material.

The soaking step may preferably be carried out under agitation. Suitable agitation may be provided by a revolving drum mixer having a heating means for heating the aqueous mixture to a desired temperature. Preferably, the axis of rotation of the revolving drum mixer is oriented about horizontally.

According to a preferred embodiment, the process may further comprise a step of centrifuging the aqueous mixture containing a plant extract and extracted plant tissue obtained in step (a). The centrifuging may conveniently be carried out in a revolving drum mixer.

Based on the soaking step, an aqueous mixture containing a plant extract and extracted plant material is obtained. Preferably, the extracted plant material is separated from the aqueous mixture. Advantageously, the aqueous mixture containing the plant extract and the extracted plant material may be filtered or decanted. The filtrate may be used as such for the following step. However, it is possible to further dilute of concentrate the aqueous mixture as the case requires.

The residue of the extracted plant material contains additional components which are not extracted in the extraction step. Accordingly, the soaking step represents a first selection and separation step in the process of the present invention. The extracted plant material may be used for further extraction in order to recover the additional components which are not extracted in the extraction step.

The process of the present invention further comprises a foaming step. Accordingly, the process of the present invention comprises a step of generating a turbulent flow condition in the aqueous mixture containing a plant extract at a temperature of from 5°C to 50°C in the presence of a gaseous fluid for obtaining a foam layer containing a plant foam extract. Preferably, the process of the present invention uses only surface active agents contained in the aqueous mixture containing a plant extract, which were extracted from the plant material in the soaking and optional centrifuging step.

The foaming may be carried out by any technique which is suitable for generating a turbulent flow condition generating a foam in the presence of a gaseous fluid. However, according to a preferred embodiment, the turbulent flow condition is generated by introducing an aqueous mixture containing a plant extract as a jet of fluid into the aqueous mixture containing a plant extract. Preferably, the jet of fluid is introduced through a nozzle.

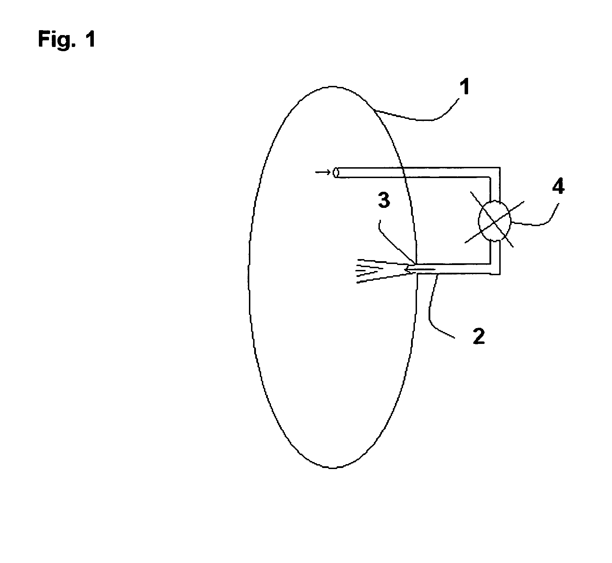

Figure 1 shows a preferred device for carrying out the foaming step in the process of the present invention. Accordingly, the device comprises a vat 1 having a inlet orifice 3 and an outlet orifice 5. The inlet orifice 3 and the outlet orifice 5 are in fluid communication through a tubing means 2. The device further comprises a pump 4 for accelerating liquid received from the outlet orifice 5 to the inlet orifice 3 as indicated by the arrows. Preferably, the liquid is accelerated to a velocity at the outlet orifice of at least 1 m/s. The inlet orifice 3 is preferably provided close to the surface level of the liquid in the vat and at least partially submerged in the liquid. Preferably, the inlet orifice is positioned at a central position of the long axis of the vat so as to provide lateral portions in the vat where the foam may accumulate. The inlet orifice 3 is preferably provided with a nozzle. It is possible use a vat having more than one inlet orifices 3 or outlet orifices in order modulate the foaming.

The foaming step is carried out at a temperature of from 5°C to 50°C. Preferably, the foaming step is carried out at a temperature of from 20 to 40°C.

The foaming step is carried out in the presence of a gaseous fluid. The gaseous fluid is conveniently air. However, in order to protect oxidizable components in the plant extract from being oxidized, it is possible to use any suitable inert gases such as nitrogen.

According to a preferred embodiment, the turbulent flow condition is generated by recycling the aqueous mixture containing a plant extract as a jet of fluid. The aqueous mixture containing a plant extract is introduced into the aqueous mixture containing the plant extract by using a nozzle. Preferably, the jet of fluid has a kinetic energy per liter of at least 1 Nm/L at the exit of the outlet orifice of the nozzle. More preferably, the jet of fluid has a kinetic energy per liter of at least 2 Nm/L for obtaining a foam layer containing a plant foam extract

According to a still more preferred embodiment, the turbulent flow condition is generated by introducing an aqueous mixture containing a plant extract as a jet of fluid into the aqueous mixture containing a plant extract, wherein the jet of fluid has an average velocity at the outlet orifice of the nozzle of at least 1 m/s. More preferably, the jet of fluid has a average velocity of at least 2 m/s.

Preferably, the outlet orifice of the nozzle has a diameter in the range of from 5 to 20 mm.

The residue of the aqueous mixture containing a plant extract contains additional components which were not transferred into the foam during the foaming step. Accordingly, the foaming step represents a second selection and separation step in the process of the present invention.

The process of the present invention further comprises a step of separating the foam layer obtaining the plant foam extract from the aqueous mixture. The foam layer may be separated batchwise or continuously as the case requires.

The process of the present invention further comprises a step of drying the separated foam layer for obtaining a plant spumate composition.

In case of the rosemary spumate composition of the present invention, it is preferred that the concentration of carnosic acid is at least 300% as compared to the concentration of carnosic acid in the starting plant material as determined by HPLC.

Moreover, the rosemary spumate composition of the present invention preferably contains the following main components in an amount as determined by headspace-SPME-GC-FID in the indicated below (more preferred range)

| compound | RI | headspace-SPME-GC-FID (Indication in %, average of two measurements) |
|---|---|---|
| 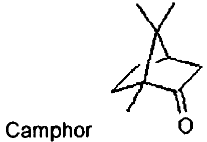 | 1139 | 45-60 (47-55) |
| 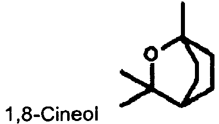 | 1027 | 10-20 (13-18) |
| 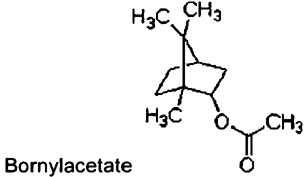 | 1281 | 3-10 (4-8) |
| 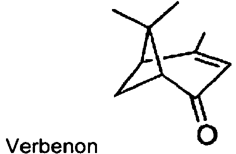 | 1202 | 1-6 (2-5) |
| 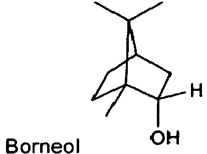 | 1161 | 1-6 (2-5) |

The plant spumate composition according to the present invention may be used for the preparation of a cosmetic composition. Representative cosmetic compositions include, in particular, those which are provided in the form of fluid emulsions (milks), lotions or in the form of more viscous emulsions (creams).

These cosmetic compositions are, for example, anti-ageing milks or creams, emollient milks or creams, milks or creams for the care of the hands, make-up remover creams or milks, foundation products for the complexion, sun screen milks or creams, artificial tanning milks or creams, anti perspirant milks or creams, and shaving creams of foams.

The cosmetic compositions of the present invention preferably contain the plant extract in a concentration between 0.01 and 10%, more preferentially between 0.1 and 5%, still more preferably 0.5 to 3% by weight based on the cosmetic composition.

When the compositions are provided in the form of creams or milks they are more particularly provided in the form of an emulsion of the water-in-oil or oil-in-water type, of which the oily phase represents from 4 to 70 percent by weight, the water phase from 30 to 96 percent by weight and the emulsifying agent from 1 to 20 percent by weight, preferably from 2 to 12 percent by weight.

The oily phase may comprise any oily component useful or conventional in the cosmetic field for this purpose. Specific examples are fats and oils including cacao butter, coconut oil, horse fat, hardened coconut oil, palm oil, beef tallow, mutton tallow, hardened beef tallow, palm kernel oil, lard, beef bone tallow, Japanese core wax, hardened oil, neatsfoot tallow, Japanese wax, and hardened castor oil. Furthermore, the oily phase may also comprise waxes including bees wax, candelilla wax, cotton wax, carnauba wax, bayberry wax, tree wax, whale wax, montan wax, bran wax, lanolin, kapok wax, lanolin acetate, liquid lanolin, sugar cane wax, lanolin fatty acid isopropyl ester, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether. Furthermore, the oily phase may also comprise synthetic ester oils or natural ester oils. As synthetic ester oils, isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoiso stearate, neopentyl glycol dicaprate, glycerol di-2-heptyl undecanoate, diisostearyl malate, trimethyrol propane tri-2-ethyl hexanoate, tetra-2-ethyl hexanoate pentaerythritol, trimethyrol propane triisostearate, glycerol tri-2-ethyl hexanoate, glycerol trioctanoate, glycerol triisopalmitate, trimethyrol propane triisostearate, cetyl 2-ethylhexanoate, 2-ethylhexyl palmitate, glycerol trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate and triethyl citrate may be mentioned. As natural ester oils, avocado oil, camellia oil, turtle oil, macadamia nut oil, corn oil, mink oil, olive oil, rapeseed oil, egg yolk oil, sesame oil, wheat germ oil, southern piece oil, castor oil, linseed oil, safflower oil, cotton seed oil, perilla oil, soybean oil, groundnut oil, torreya oil, rice bran oil, chinese wood oil, jojoba oil, germ oil, triglycerol, trioctane acid glycerol and triiso palmitic acid glycerol may be mentioned. Moreover, the oily phase may also comprise higher fatty acids including lauric acid, myristic acid, palmitic acid, stearic acid, behenic acid, oleic acid, undecylenic acid, isostearic acid, linoleic acid, linolenic acid, eicosapentaenoic acid (EPA), and docosahexaenoic acid (DHA).

Representative emulsifying agents include the plant spumate composition according to the present invention, and other emulsifying agents, for instance: Glycerol mono fatty acid esters, for example the product sold under the name of Tegomuls 90S by Th. Goldschmidt, glycerolpolyoxyethylenated or polyglycerolated fatty alcohols, alkyl sulfates oxyethylenated or not, mixtures of at least one lanolate such as magnesium, calcium, lithium, zinc or aluminum lanolate and hydrogenated lanolin and/or lanolin alcohol, esters of fatty acids and polyols such as glycerol or propylene glycol; and monoesters of fatty acids and polyoxyelthylenated sorbitan, for example, the product sold under the trade name TWEEN by Atlas.

The cosmetic compositions of the present invention can also contain other conventional components including thickening agents or gelling agents such as, for example, magnesium silicate, aluminum silicate, vinyl ether/maleic anhydride copolymers, such as products sold under the tradenames VISCOFAS X.100.000 or VISCOFAS L.100 by ICI associated with amino-acids, or even carboxyvinyl polymers such as the products sold under the trade name CARBOPOL by Goodrich.

The cosmetic compositions according to the present invention can also contain various adjuvants such as dyes, perfumes, preservatives, UV filters, pigments, nacreous agents and mineral or organic charges or fillers.

### Examples

The present invention will now be illustrated based on the following examples. Unless otherwise specified, all parts and percentages are by weight.

### REFERENCE EXAMPLE 1

### Analysis of rosemary starting material.

1 kg of wild rosemary starting material consisting essentially of leaves and flowers was collected and comminuted. The essential oil was extracted from the comminuted rosemary starting material by vapor distillation and the resulting substance analyzed by gas chromatography (GC). The components of the essential oil were identified based on reference compounds.

The following composition was found:

| Compound | RI | % |
|---|---|---|
| DMS | 598 | <0,1 |
| 3-methylbutanal | 649 | <0,1 |
| 2-methylbutanal | 651 | <0,1 |
| tricyclene | 918 | 0,4 |
| alpha-thujene | 923 | 0,2 |
| alpha-pinene | 931 | 17,2 |
| camphene | 944 | 9,4 |
| thuja-2,4(10)dien | 948 | 0,3 |
| beta-pinene | 972 | 4,4 |
| 1-octen-3-ol | 975 | 0,2 |
| 3-octanon | 982 | 1,2 |
| 2,3-dehydro-1,8-cineol | 988 | <0,1 |
| myrcene | 989 | 3,5 |
| alpha-phellandrene | 1002 | 0,9 |
| delta-3-carene | 1007 | 0,1 |
| alpha-terpinene | 1013 | 0,5 |
| p-cymol | 1021 | 3,0 |
| limonene | 1026 | 2,0 |
| 1,8-cineol (eucalyptol) | 1027 | 23,4 |
| Z-ß-ocimen | 1035 | 0,2 |
| phenylacetaldehyd | 1045 | <0,1 |
| gamma-terpinene | 1054 | 0,9 |
| trans-sabinenhydrat | 1062 | <0,1 |
| terpinolene | 1084 | 0,4 |
| cis-sabine hydrate | 1094 | <0,1 |
| Linalool | 1098 | 0,6 |
| n.i. m/e 80,107,150 | 1110 | 0,1 |
| fenchol | 1119 | 0,1 |
| p-menth-2-en-1-ol A | 1120 | <0,1 |
| chrysanthenon ? | 1126 | <0,1 |
| p-menth-2-en-1-ol B | 1133 | <0,1 |
| camphor | 1139 | 18,0 |
| Camphene hydrate | 1142 | <0,1 |
| delta-terpineol | 1155 | 0,2 |
| borneol | 1161 | 3,1 |
| terpinen-4-ol | 1172 | 0,7 |
| alpha-terpineol | 1186 | 1,0 |
| myrtenol | 1197 | 0,0 |
| verbenone | 1202 | 2,1 |
| n.i | 1230 | 0,1 |
| n.i. | 1235 | 0,1 |
| piperitone | 1246 | <0,1 |
| Bornyl acetate | 1281 | 1,3 |
| thymol | 1285 | <0,1 |
| carvacrol | 1293 | <0,1 |
| piperitenon | 1332 | <0,1 |
| eugenol | 1352 | <0,1 |
| alpha-ylangene | 1366 | 0,1 |
| alpha-copaene | 1371 | <0,1 |
| E-beta-caryophyllene | 1414 | 2,0 |
| n.i. m/e 105,161,204, | 1438 | <0,1 |
| alpha-humulene | 1448 | 0,6 |
| gamma-muurolene | 1472 | <0,1 |
| alpha-amorphene | 1475 | 0,1 |
| germacrene D | 1479 | <0,1 |
| ß-bisabolene | 1489 | <0,1 |
| delta-amorphene | 1502 | 0,1 |
| gamma-cadinene | 1505 | <0,1 |
| delta-cadinene | 1508 | <0,1 |
| calamenene | 1519 | 0,1 |
| alpha-Calacorene | 1536 | <0,1 |
| Caryophyllene oxide | 1575 | 0,3 |
| alpha-humulen-1,2-epoxid | 1601 | 0,1 |

The distribution of the components corresponds to a typical composition of a rosemary oil originating from Spain (cf. Kubeczka, K.H. Essential Oil Analysis by Capillary Gas Chromatography and Carbon-13-NMR Spectroscopy, page 285f: "Rosemary Oil spain type: alpha-Pinen 17,74 %, 1,8-Cineol 22,97 %, Camphor 18,54 %)

Moreover, the rosemary starting material was extracted with water for 60 minutes at pH 7.0. HPLC-Analysis of the aqueous solution indicated a carnosolic acid concentration of the rosemary starting material of 305mg/kg and a carnosol concentration of 12mg/kg, whereby carnosol is believed to be derived from carnosolic acid under the extraction conditions. Given a carnosolic acid concentration of 240-250 mg/kg in conventionally known rosemary starting material, the specific rosemary starting material used has a superior antioxidant composition.

### EXAMPLE 1

### Soaking of a rosemary herbal material in water

4.0 kg of rosemary herbal material consisting essentially of leaves and flowers was introduced into a front loading revolving drum mixer having a horizontal rotational axis, a drum capacity of 70 liters and being equipped with a heating means.

Subsequently, the lid of the mixing device was sealed and 25 liters of tab water having a pH of 7.0 was introduced into the revolving drum mixer through an inlet valve. The content of the mixer was agitated by revolving the drum at 20 rpm while the water was heated to a temperature of 60 °C. Subsequently, the rosemary herbal material was soaked under agitation at 60 °C for 60 minutes for obtaining an aqueous mixture containing a rosemary herbal extract and extracted herbal material. Subsequently, the revolving drum was stopped and heating was discontinued. The extracted herbal material was separated from the aqueous mixture by decanting for obtaining 25 liters of aqueous mixture containing a rosemary herbal extract.

The process step was repeated four times in order to provide a total amount of 100 liters of aqueous mixture containing a rosemary herbal extract

### EXAMPLE 2

### Foaming of an aqueous mixture containing a rosemary herbal extract

In a vat having an oval cross section with a short diameter of 75 cm, and a nozzle in the center section of a long side as shown in Figure 1, 100 liters of the aqueous mixture containing a rosemary herbal extract were placed. The vat is equipped with an outlet for removing liquid from the vat. The outlet is in fluid flow communication with a pump and the nozzle for recycling the liquid as a jet of fluid into the vat. The nozzle has an orifice having a diameter of 13 mm, and directs the jet of fluid parallel to the upper surface of the aqueous mixture containing a rosemary herbal extract. The nozzle orifice is partly submerged in the aqueous mixture. The pump is adjusted so that the jet of fluid has an average velocity at the outlet orifice of the nozzle of about least 2 m/s. Accordingly, the jet of fluid introduced into the liquid has a kinetic energy per liter of 2 Nm.

Subsequently a turbulent flow condition is generated in the aqueous mixture containing a herbal extract at a temperature of from 30°C by recycling the aqueous mixture as a jet of fluid into the vat. The initially formed foam is unstable and cannot be harvested so that the turbulent flow condition must be maintained for about 10 minutes until a stable creamy foam of a light yellow color is formed. The foaming is continued for 60 minutes before for obtaining a foam layer containing a herbal foam extract. The foam layer containing a stiff and stable foam is then separated from the aqueous mixture by using a sieve. Subsequently, the foaming is repeated for a total of four times. The foam layers obtained are combined and dried overnight in a dry athmosphere for providing 25.5 g of a herbal spumate composition as a light particulate substance having a pleasant rosemary odor.

### EXAMPLE 3

### (i) Analysis of antioxidants in the herbal spumate composition using HPLC

HPLC was used to determine the content of carnosic acid in the rosemary spumate. The concentration was determined to be 1250 mg/Kg with a standard deviation of 35. It was found that this amount corresponds to a fourfold entrichment as compared to the natural content of this component in the rosemary plant. The amount of carnesol was determined to be 62 mg/Kg which is fivefold higher as compared to the natural content of this component in the rosemary plant.

### (ii) Analysis of the herbal spumate composition using Headspace-SPME-GC-MS

Quantitative analysis was carried out by using headspace-SPME-GC-FID (Indication in %, average of two measurements), headspace-SPME-GC-MS was also used for the identification by comparison of mass spectra und retention indices (RI) with a reference library and literature data.
GC: sample weight 50 mg, Column: DVB-CAR-PDMS (grey), enrichment 30 min, 25 °C
GC-MS: sample weight 30 mg, Column: DVB-CAR-PDMS (grey), enrichment 30 min, 25 °C

| Component | RI | % |
|---|---|---|
| α-Thujene | 923 | 0,09 |
| α-Pinene | 931 | 0,75 |
| Camphene | 944 | 0,58 |
| Thuja-2,4(10)-dien | 948 | 0,13 |
| ß-Pinene | 972 | 0,57 |
| 1-Octen-3-ol | 975 | 0,06 |
| 3-Octanon | 982 | 0,72 |
| Myrcene | 989 | 0,58 |
| α-Phellandrene | 1002 | 0,18 |
| α-Terpinene | 1013 | 0,11 |
| p-Cymol | 1021 | 2,91 |
| Limonen | 1026 | 1,63 |
| 1,8-Cineol | 1027 | 15,07 |
| γ-Terpinen | 1054 | 0,18 |
| Artemisiaketon | 1058 | 0,08 |
| trans-Sabinenhydrat | 1062 | 0,05 |
| Terpinolen + p-Cymenen | 1084 | 0,24 |
| Linalool | 1098 | 0,90 |
| n.i. m/e 80,107,150 | 1110 | 0,09 |
| Fenchol | 1119 | 0,05 |
| m/e 107,108,150 | 1120 | 0,09 |
| Camphor | 1139 | 53,31 |
| Pinocarvone | 1153 | 0,40 |
| δ-Terpineol ? | 1155 | 0,85 |
| Borneol | 1161 | 3,38 |
| iso-Pinocamphone | 1167 | 1,28 |
| Terpinen-4-ol | 1172 | 1,15 |
| n.i. | 1181 | 0,25 |
| α-Terpineol | 1186 | 0,73 |
| Myrtenol | 1189 | 0,08 |
| Myrtenal | 1192 | 0,11 |
| Verbenone | 1202 | 3,72 |
| Bornylacetat | 1281 | 6,30 |
| α-Ylangene | 1366 | 0,45 |
| α-Copaene | 1371 | 0,11 |
| E-ß-Caryophyllene | 1414 | 1,81 |
| ß-Copaene | 1425 | 0,08 |
| α-Humulene | 1448 | 0,46 |
| γ-Muurolene | 1472 | 0,11 |
| α-Amorphene | 1475 | 0,14 |
| ß-Bisabolene | 1489 | 0,11 |
| γ-Cadinene | 1505 | 0,08 |
| δ-Cadinene | 1508 | 0,08 |
| Calamenene | 1519 | 0,08 |
| α-Calacorene | 1536 | 0,02 |

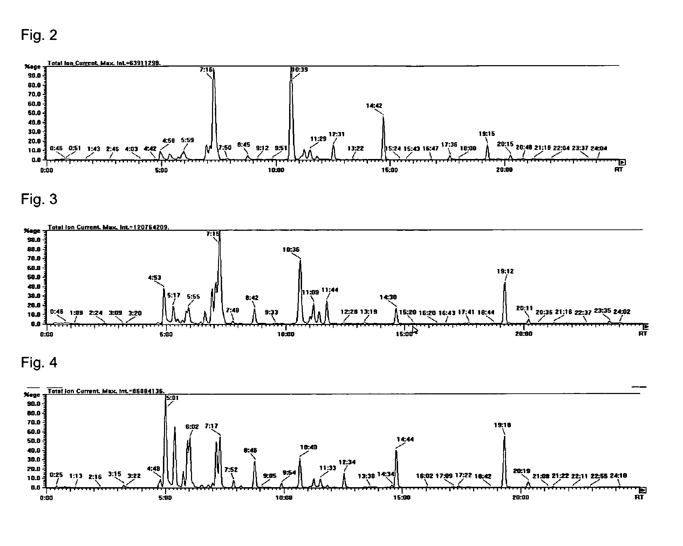

For purposes of a comparison, commercial dried rosemary and freshly cut rosemary were analyzed under the same HA-SPME-GC-MS conditions. The results are shown in figures 2 to 4.

Regarding the quantitative composition, the content of volatile monoterpene-hydrocarbons (retention time about 5 - 7 min) shows significant differences. The highest content is in freshly cut rosemary (Fig 4). In the dried rosemary sample, volatile components are lost due to the drying Step (Fig 3). The lowest content is found in the rosemary-spumate according to the present invention (Fig.2), whereby the main components are as follows:

| | |
|---|---|
| 4:58 | α-Pinene |
| 5:59 | ß-Pinene + Myrcene |
| 7:16 | 1,8-Cineol |
| 8:45 | Linalool |
| 10:39 | Camphor |
| 11:16 | Borneol |
| 12:31 | Verbenon |
| 14:42 | Bornyl acetate |
| 17:36 | α-Ylangene |
| 19:15 | ß-Caryophyllene |
| 20:15 | α-Humulene |

The analysis shows that the rosemary spumate composition according to the present invention has a significantly higher antioxidative potential as compared to the rosemary plant whereas the amount of aromatic substances is significantly reduced.

### EXAMPLE 4

### Antioxidant properties of a rosemary herbal spumate composition

150 g of freshly crushed avocado fruit pulp (Persea americana) was divided into two test beakers. 0.5 g of the herbal spumate composition was dispersed in the crushed avocado fruit pulp in a first beaker for providing a test composition. The macerated avocado fruit pulp in the second beaker was used as a control. The beakers were stored at 25° C in the air.

As a result, it was found that the crushed avocado fruit pulp of the control continuously changed color from yellowish green to brown over a period time of 2 days before spoiling. On the other hand, the test example remained stable in color for more than 6 weeks indicating a high storage stability as compared to the control.

### EXAMPLE 5

### Soaking of hypericum (St John's wort) herbal plant material in water

4.0 kg of leafs and fruits of hypericum (St John's wort) was introduced into a front loading revolving drum mixer having a horizontal rotational axis, a drum capacity of 70 liters and being equipped with a heating means.

Subsequently, the lid of the mixing device was sealed and 25 liters of tab water having a pH of 7.0 was introduced into the revolving drum mixer through an inlet valve. The content of the mixer was agitated by revolving the drum at 20 rpm while the water was heated to a temperature of 60 °C. Subsequently, the rosemary herbal material was soaked under agitation at 60 °C for 60 minutes for obtaining an aqueous mixture containing a rosemary herbal extract and extracted herbal material. Subsequently, the revolving drum was stopped and heating was discontinued. The extracted herbal material was separated from the aqueous mixture by decanting for obtaining 25 liters of aqueous mixture containing a rosemary herbal extract.

The process step was repeated four times in order to provide a total amount of 100 liters of aqueous mixture containing a rosemary herbal extract

### EXAMPLE 6

### Foaming of an aqueous mixture containing a hypericum herbal extract

In a vat having an oval cross section with a short diameter of 75 cm, and a nozzle in the center section of a long side as shown in Figure 1, 100 liters of the aqueous mixture containing a rosemary herbal extract were placed. The vat is equipped with an outlet for removing liquid from the vat. The outlet is in fluid flow communication with a pump and the nozzle for recycling the liquid as a jet of fluid into the vat. The nozzle has an orifice having a diameter of 13 mm, and directs the jet of fluid parallel to the upper surface of the aqueous mixture containing a rosemary herbal extract. The nozzle orifice is partly submerged in the aqueous mixture. The pump is adjusted so that the jet of fluid has an average velocity at the outlet orifice of the nozzle of about least 2 m/s. Accordingly, the jet of fluid introduced into the liquid has a kinetic energy per liter of 2 Nm.

Subsequently a turbulent flow condition is generated in the aqueous mixture containing a herbal extract at a temperature of from 30°C by recycling the aqueous mixture as a jet of fluid into the vat. The initially formed foam is unstable and cannot be harvested so that the turbulent flow condition must be maintained for about 10 minutes until a stable creamy foam of a light yellow color is formed. The foaming is continued for 60 minutes before for obtaining a foam layer containing a herbal foam extract. The foam layer containing a stiff and stable foam is then separated from the aqueous mixture by using a sieve. Subsequently, the foaming is repeated for a total of four times. The foam layers obtained are combined and dried overnight in a dry athmosphere for providing a herbal spumate composition.

### EXAMPLE 7

### Preparation of a skin cosmetic cream using a rosemary spumate composition

A solution of 3 % by weight based on the total weight of the solution of the rosemary spumate composition obtained according to Example 2 in 97% by weight based on the total weight of the solution of a hydrolate of was prepared and any filtered for providing a clear rosemary spumate solution. The hydrolate was obtained as an aqueous solution or colloidal suspension (hydrosol) of essential oils obtained by steam distillation from Rosmarinus officinalis L., Juniperus oxycedrus and Pistacia lentiscus.

A cream was prepared by combining and mixing the following components at 60 °C in the following amounts by weight based on the entire weight of the cream for obtaining a cosmetic composition of the invention which is useful as an anti-ageing cream.

| | |
|---|---|
| Rosemary spumate solution | 43 |
| Olive oil | 14 |
| Avocado (Persea americana) | 16 |
| Aloe Vera | 8 |
| Glycerol monostearate | 5 |
| Cetyl alcohol | 6 |
| Perfume | balance |

## Claims

1. Process for the preparation of a plant spumate composition, comprising
(a) soaking plant material in water for a predetermined amount of time at a temperature of from 40°C to 100°C for obtaining an aqueous mixture containing a plant extract and extracted plant material;
(b) generating a turbulent flow condition in the aqueous mixture containing a plant extract at a temperature of from 5°C to 50°C in the presence of a gaseous fluid for obtaining a foam layer containing a plant foam extract;
(c) separating the foam layer from the aqueous mixture; and
(d) drying the separated foam layer for obtaining a plant spumate composition.

2. The process according to claim 1 wherein the turbulent flow condition is generated by introducing an aqueous mixture containing a plant extract as a jet of fluid into the aqueous mixture containing a plant extract.

3. The process according to claim 1 or 2, wherein the turbulent flow condition is generated by introducing an aqueous mixture containing a plant extract as a jet of fluid into the aqueous mixture containing a plant extract, wherein the jet of fluid has a kinetic energy per liter of at least 1 Nm.

4. The process according to any one of claims 1 to 3, wherein the turbulent flow condition is generated by introducing an aqueous mixture containing a plant extract as a jet of fluid into the aqueous mixture containing a plant extract, wherein the jet of fluid has an average velocity at the outlet orifice of the nozzle of at least 1 m/s.

5. The process according to any one of claims 1 to 4, wherein the turbulent flow condition is generated by introducing an aqueous mixture containing a plant extract as a jet of fluid into the aqueous mixture containing a plant extract, wherein the outlet orifice has a diameter in the range of from 5 to 20 mm.

6. The process according to any one of the preceding claims, which further comprises a step of centrifuging the aqueous mixture containing a plant extract and extracted plant tissue obtained in step (a) for obtaining a concentrated aqueous mixture containing a plant extract.

7. The process according any one of the preceding claims wherein the plant material includes leaves and/or flowers, preferably consist of leaves and/or flowers.

8. The process according to any one of the preceding claims, wherein the plant material is used without being comminuted.

9. The process according to any one of the preceding claims, wherein the soaking step is carried out under agitation.

10. The process according to any one of the preceding claims, wherein the soaking is carried out for 20 to 90 minutes, preferably 40 to 60 minutes.

11. The process according to any one of the preceding claims which further comprises a step of comminuting the plant spumate composition.

12. The process according to any one of the preceding claims, wherein the plant is selected from the group of from Rosemary *(Rosmarinus officialis)* containing carnosic acid((4aR,1 0aS)-5,6-dihydroxy-1,1-dimethyl-7-propan-2-yl-2,3,4,9,10,10a-hexahydrophenanthrene-4a-carboxylic acid); hypericum (St John's wort) containing hyperforin ((1R,5S,7R,8S)-4-hydroxy-8-methyl-3,5,7-tris(3-methylbut-2-enyl)-8-(4-methylpent-3-enyl)-1-(2-methylpropanoyl)bicyclo[3.3.1]non-3-ene-2,9-dione); chili pepper and other plants of the genus *Capsicum* containing capsaicin (8-Methyl-N-vanillyl-trans-6-nonenamide); ginger containing gingerole ((S)-5-hydroxy-1-(4-hydroxy-3-methoxyphenyl)-3-decanone); hops containing humulone ((6R)-3,5,6-Trihydroxy-2-(3-methylbutanoyl)-4,6-bis(3-methylbut-2-en-1-yl)cyclohexa-2,4-dien-1-one); orange (Citrus sinensis) containing xanthophylls including lutein, zeaxanthin, neoxanthin, violaxanthin, and α- and β-cryptoxanthin; carrots (Daucus carota subsp. Sativus) containing carotene; curcuma containing curcuminoide (1 E,6E)-1,7-bis (4-hydroxy-3-methoxyphenyl) -1,6-heptadiene-3,5-dione; Potato fruits (Solanum tuberosum)containing solanine; kava plant containing Flavokavin (A: (E)-3-(4-methoxyphenyl)-1-(2,4-bismethoxy-6-hydroxyphenyl)propenone, B: (E)-1-(2-Hydroxy-4,6-dimethoxy-phenyl)-3-phenyl-propenone, C: (E)-1-(2-Hydroxy-4,6-dimethoxy-phenyl)-3-(4-hydroxy-phenyl)-propenone); plants of the genus Coffea containing chlorogenic acid (1 S,3R,4R,5R)-3-{[(2Z)-3-(3,4-dihydroxyphenyl)prop-2-enoyl]oxy}-1,4,5-trihydroxycyclohexanecarboxylic acid; plants of the genus Sesamum containing sesamole (3,4-Methylenedioxyphenol); plants of the family Piperaceae such as black pepper (Piper nigrum) which contain piperine (1-[5-(1,3-benzodioxol-5-yl)-1-oxo-2,4-pentadienyl]piperidine); Aloe vera containing aloin (e.g. (10S)-10-Glucopyranosyl-1,8-dihydroxy-3-(hydroxymethyl)-9(10H)-anthracenone); onion (Allium cepa) containing isoalliin; and Prunus dulcis containing amygdalin [(6-O-β-D-glucopyranosyl-β-D-glucopyranosyl)oxy](phenyl)acetonitrile is rosemary. (*Rosmarinus officinalis*), preferably wherein the rosemary has a content of carnosic acid of at least 260 mg/kg, preferably at least 280 mg/kg.

13. A plant spumate composition obtainable by the process of any one of the preceding claims.

14. A cosmetic composition containing the plant spumate composition according to claim 13, preferably which is a skin cream or a facial mask.

15. Use of a plant spumate composition according toclaim 13 for the preparation of a cosmetic composition, preferably wherein the cosmetic composition is as defined in claims 14.
